# EUROPÄISCHE PATENTSCHRIFT

(11) **EP 0 624 379 B1**
(45) Veröffentlichungstag und Bekanntmachung des Hinweises auf die Patenterteilung: **25.07.2001**
(21) Anmeldenummer: 94107037.7
(22) Anmeldetag: 05.05.1994
(51) Int. Cl.: A61M 5/142, F04B 3/00, F04B 9/02

(54) **Doppelkolbenpumpe für medizinische Anwendungen**
Double acting piston pump for medical use
Pompe à piston à double effet pour usage médical

(30) Priorität: 10.05.1993 CH 143293
(43) Veröffentlichungstag der Anmeldung: 17.11.1994
(73) Patentinhaber: MEDIMPEX ETS., FL-9496 Balzers (LI)
(72) Erfinder: Huggenberger, Heinz, CH-3308 Grafenried (CH)
(74) Vertreter: Lusuardi, Werther Giovanni, Dr.

(56) Entgegenhaltungen:
- US-A- 3 791 770
- US-A- 4 221 548
- US-A- 4 273 122
- US-A- 4 838 860

## Beschreibung

Die Erfindung betrifft eine Vorrichtung gemäss dem Oberbegriff des Patentanspruchs 1.

Aus der DE-OS 40 16 306 ist bereits eine Radialkolbenpumpe zur Förderung von kleinen Flüssigkeitsmengen bekannt, bei welcher zwei unabhängige Pumpenkolben gegenläufig bewegt werden und über gesteuerte Ventile die Flüssigkeitsförderung bewirken. Die Nachteile dieser bekannten Radialpumpe liegen in den konstruktionsbedingt auftretenden hohen Querkräften und der damit verbundenen Reibung sowie den hohen Anforderungen an die Herstellungspräzision der beiden zusammenwirkenden Radialscheiben.

Eine Infusionspumpe zur Förderung einer Flüssigkeit aus einem Flüssigkeitsreservoir zu einem Patienten ist aus der US 4,838,860 GROSHONG bekannt. Diese bekannte Kolbenpumpe umfasst zwei an den Enden einer oszillierenden Kolbenstange angebrachte Kolben, an die Zylinderkammern anschliessend zwei Kanäle zur Flüssigkeitszufuhr respektive -abfuhr, welche in einer gewissen Entfernung von den Zylinderkammern in separate Flüssigkeitszu- und abfuhrleitungen aufgeteilt werden, und vier als Ventile dienende, deformierbare Röhrchen, welche durch einen Schieber schliessbar sind. Der Schieber bewirkt, dass in seiner einen Stellung die von der linken Zylinderkammer wegführende Flüssigkeitsabführleitung und die zur rechten Zylinderkammer hinführende Flüssigkeitszuführleitung geöffnet sind während gleichzeitig die zur linken Zylinderkammer hinführende Flüssigkeitszufuhrleitung und die von der rechten Zylinderkammer wegführende Flüssigkeitsabführleitung geschlossen werden und in seiner anderen Stellung die als Ventile dienenden, deformierbaren Röhrchen so geschlossen, beziehungsweise geöffnet sind, dass der linken Zylinderkammer Flüssigkeit zugeführt wird während von der rechten Zylinderkammer Flüssigkeit abgeführt wird. Nachteilig bei dieser bekannten Pumpe ist, dass die als Ventile dienenden, deformierbaren Röhrchen werden durch den Schieber zwangsweise gesteuert.

Hier will die Erfindung Abhilfe schaffen. Der Erfindung liegt die Aufgabe zugrunde, eine Vorrichtung zur Abgabe einer vorwählbaren Dosis eines flüssigen Medikamentes zu schaffen, welche einfach zu handhaben ist, eine präzise Ausschüttung gestattet und eine Variation der Medikamentendosis zulässt.

Die Erfindung löst die gestellte Aufgabe mit einer Vorrichtung, welche die Merkmale des Anspruchs 1 aufweist.

Die durch die Erfindung erreichten Vorteile sind im wesentlichen die folgenden:
- Dank des erfindungsgemässen Doppelkolbens tritt nie ein Unterdruck im System auf; es besteht im Betriebszustand ein ständiger Medikamentenfluss;
- dank der getrennten Ausführung von Pumpeneinheit und Antriebseinheit, die in einfacher Weise zusammenklickbar sind, ergibt sich eine sehr einfache, patientenfreundliche Handhabung;
- durch die gespritzten Kunststoffteile der Pumpeneinheit wird die Ausschüttungsqualität durch die Pumpe selbst und nicht durch äussere Parameter, wie Druck, Temperatur, Feuchtigkeit u.ä. bestimmt;
- die Variation der Medikamentendosis kann durch die Parameter der Pumpeneinheit (Kolbendurchmesser) und Steigung des Gewindes der Kolbenstange vorgegeben werden; auf diese Weise lässt sich eine entsprechende Normreihe von Pumpeneinheiten aufbauen;
- die Antriebseinheit kann für längere Zeit verwendet werden, d.h. sie kann für mehrere Medikamentenbehälter bei einem Patienten eingesetzt werden;
- die Pumpeneinheit ist als Einwegsystem zum einmaligen Gebrauch konzipiert; und
- die Vorrichtung ist gegen eine patientenseitige Entnahme von Medikamentenflüssigkeit aus dem Medikamentenbehälter gesichert.

Ein Ausführungsbeispiel der Erfindung, welches zugleich das Funktionsprinzip erläutert, ist in der Zeichnung dargestellt und wird im folgenden näher beschrieben.
Fig. 1 stellt eine schematische Darstellung der Vorrichtung gemäss der Erfindung dar;
Fig. 2 zeigt ein modifiziertes Detail der Vorrichtung nach Fig. 1; und
Fig. 3 zeigt einen Querschnitt durch die Vorrichtung gemäss der Erfindung dar.

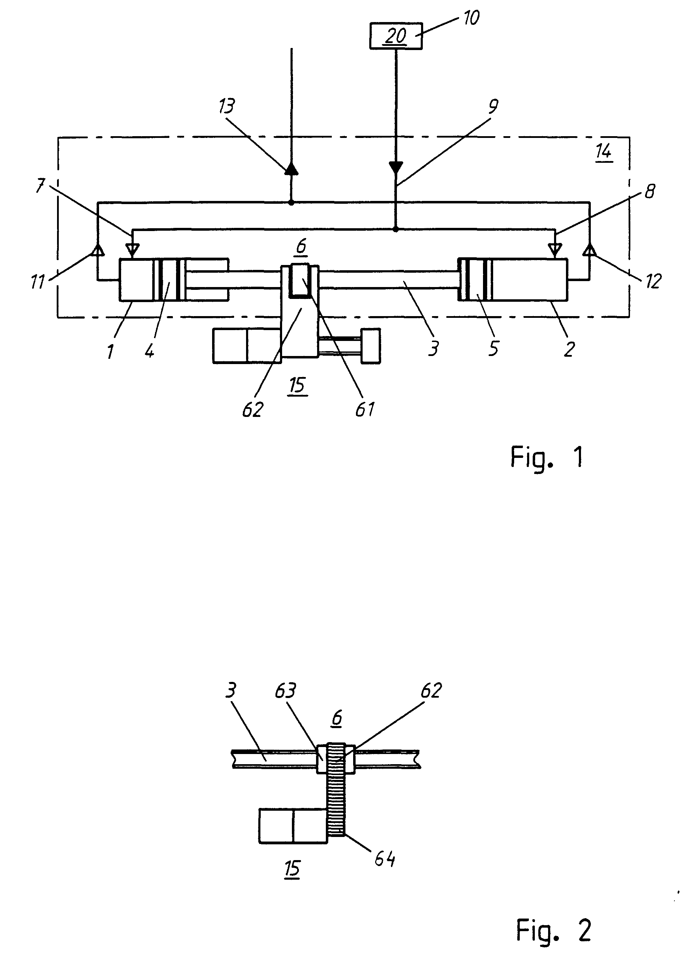

Die in den Fig. 1 - 3 dargestellte Vorrichtung zur Abgabe einer vorwählbaren Dosis eines flüssigen Medikamentes besteht im wesentlichen aus einer, mit einem Medikamentenbehälter 10 verbundenen Pumpeneinheit 14.
Die Pumpeneinheit 14 umfasst einen Zylinder 1,2 mit einem linken Zylinderteil 1 und einem rechten Zylinderteil 2 in welchem ein Doppelkolben mit einem linken Kolbenteil 4 und einem rechten Kolbenteil 5 verschieblich angeordnet ist. Der linke Kolbenteil 4 und der rechte Kolbenteil 5 sind über eine Kolbenstange 3 fest miteinander verbunden, wobei die beiden Kolbenteile 4,5 gleichsinnig in den beiden Zylinderteilen 1,2 verschieblich angeordnet sind. An der Kolbenstange 3 sind Mittel 6 vorgesehen, welche über eine Mitnehmergabel 62 an eine Antriebseinheit 15 koppelbar sind. Es ist aber auch eine Ausführung möglich, bei welcher der Zylinder 1,2 induktiv bewegt wird.

Die Steuerung der Vorrichtung erfolgt völlig passiv mittels je zweier selbstschliessender Einlassventile 7,8 und Auslassventile 11,12. Zu diesem Zweck ist für den linken Zylinderteil 1 ein linkes Einlassventil 7 und für den rechten Zylinderteil 2 ein rechtes Einlassventil 8 vorgesehen, wobei die beiden Einlassventile 7,8 über eine Einlassleitung 9 an einen Medikamentenbehälter 10 anschliessbar sind. Für den linken Zylinderteil 1 ist ein linkes Auslassventil 11 und für den rechten Zylinderteil 2 ist ein rechtes Auslassventil 12 vorgesehen, wobei die beiden Auslassventile 11,12 über eine Auslassleitung 13 das flüssige Medikament 20 abgeben. Die Auslassleitung 13 ist durch eine Durchstechmembrane 16 von der Aussenatmosphäre getrennt, durch welche ein Katheter oder eine Kanüle geführt werden kann. Analog dazu ist die Einlassleitung 9 durch eine Durchstechmembrane 17 von der Aussenatmosphäre getrennt und mittels einer Aufsteckkappe 18 gegen eine patientenseitige Entnahme von Medikamentenflüssigkeit 20 aus dem Medikamentenbehälter 10 gesichert.

Der Medikamentenbehälter 10 enthält einen Sack 19, der im Anlieferungszustand weitestgehend evakuiert ist. Um ihn für den bestimmungsgemässen Gebrauch mit Medikamentenflüssigkeit 20 aufzufüllen, genügt es mittels einer Spritze die Durchstechmembrane 17 zu perforieren und den Sack 19 über die Einlassleitung 9 aufzufüllen. Danach wird die Durchstechmembrane 17 mit der Aufsteckkappe 18 gesichert.

Die selbststeuernden Einlassventile 7,8 und die darum herumgeführten Teile der Einlassleitung 9 sind durch eine Gummimatte 23 gegenüber den beiden Körpern 22 und 22' des Zylinders 1,2 abgedichtet. Die Gummimatte 23 ist mit peripheren Dichtungsrippen versehen, welche zwar eine Verbindung zwischen den Einlassleitungen 9 gestattet, aber im übrigen den Abfluss von Flüssigkeit in andere Regionen verhindert. Ebenso sind die selbststeuernden Auslassventile 11,12 und die darum herumgeführten Teile der Auslassleitung 13 durch eine Gummimatte 21, welche gleich gestaltet ist wie die Gummimatte 23, gegenüber den Körpern 22 und 22' des Zylinders 1,2 abgedichtet. Die Einlassventile 7,8 und die Gummimatte 23 sind herstellungsmässig aus einem einzigen Stück eines elastomeren Materials gefertigt, wobei der zentrale Teil die Dichtungsfunktion der Gummimatte 23 ausübt und die Endteile die Ventilfunktion ausüben. Die Gummimatte 21 und die Auslassventile 11,12 sind in analoger Weise zusammengefügt. Die beiden Gummimatten 21 und 23 wirken somit sowohl als Dichtungen als auch als Ventile.

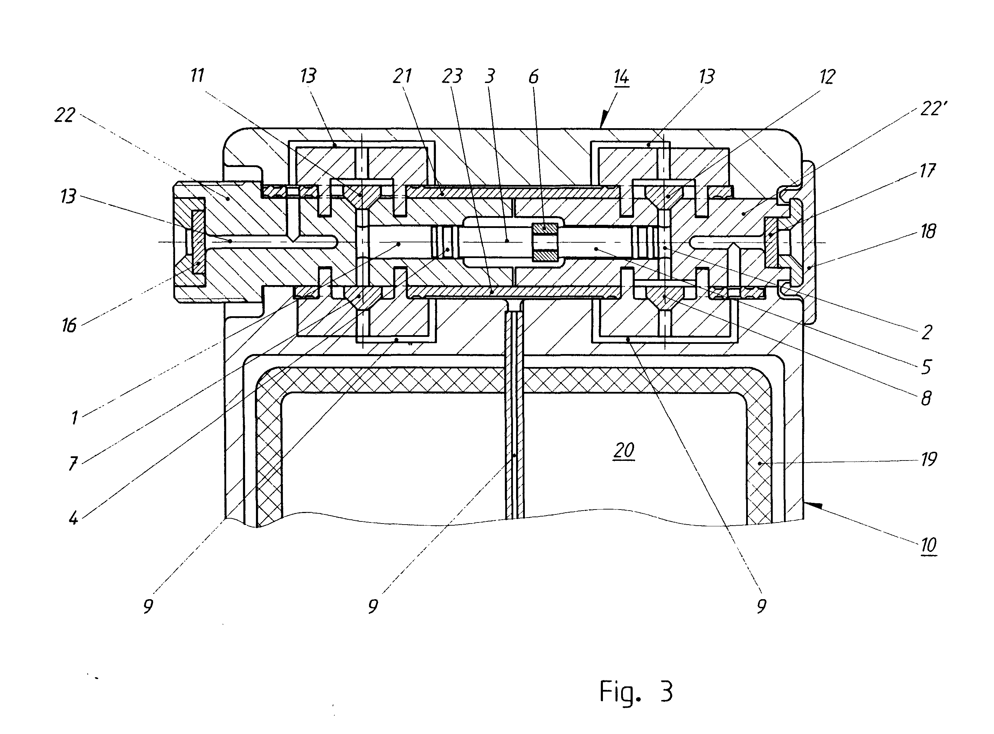

Anhand der Fig. 3 soll nun ein vollständiger Zyklus der Pumpeneinheit 14 beschrieben werden.

Der Ausgangszustand ist in Fig. 3 dargestellt, in welchem die beiden Einlassventile 7 und 8, sowie die beiden Auslassventile 11 und 12 durch die leichte Überdimensionierung des elastischen Ventil-Materials geschlossen sind. In einer ersten Phase wird der Doppelkolben 4,5 nach links bewegt. Die Bewegung des Doppelkolben 4,5 erfolgt induktiv (oder auch - wie anhand der Fig. 1 oben beschrieben - mechanisch) mit Hilfe der an der Kolbenstange 3 vorgesehenen Mittel 6. Das Auslassventil 12 bleibt weiterhin geschlossen währenddem das Einlassventil 8 durch den im rechten Zylinderteil 2 entstehenden Unterdruck geöffnet wird, so dass durch die Einlassleitung 9 Medikamentenflüssigkeit 20 aus dem Sack 19 durch die Einlassleitung 9 in den rechten Zylinderteil 2 angesaugt wird. Gleichzeitig bleibt das Einlassventil 7 durch den im linken Zylinderteil 1 erzeugten Druck und den Ansaugunterdruck in der Einlassleitung 9 weiterhin geschlossen währenddem das Auslassventil 11 geöffnet wird, so dass die im linken Zylinderteil 1 allfällig vorhandene Luft über die Auslassleitung 13 (durch einen die Durchstechmembrane 16 perforierenden Katheter) ausgestossen wird.

In einer zweiten Phase wird der Doppelkolben 4,5 - nach Erreichen seiner linken Endstellung - wider nach rechts bewegt. Dadurch wird das Auslassventil 11 geschlossen und das Einlassventil 7 geöffnet, so dass Medikamentenflüssigkeit 20 aus dem Sack 19 über die Einlassleitung 9 in den linken Zylinderraum 1 angesaugt wird. gleichzeitig wird das Einlassventil 8 geschlossen und das Auslassventil 12 geöffnet, so die in der ersten Phase in den rechten Zylinderraum 2 angesaugte Medikamentenflüssigkeit 20 durch die Auslassleitung 13 - am geschlossenen Auslassventil 11 vorbei - (durch einen die Durchstechmembrane 16 perforierenden Katheter) ausgestossen wird.

Nach Erreichen seiner rechten Endstellung wird der Doppelkolben 4,5 wieder nach links bewegt, so dass sich der Ablauf der ersten Phase wiederholt, mit dem einzigen Unterschied, dass nun keine Luft mehr aus dem linken Zylinderraum 1, sondern Medikamentenflüsssigkeit 20 ausgestossen wird.
Auf diese Weise wird durch die gleichsinnige Recht-Links-Bewegung des Doppelkolbens 4,5 ein wiederholter Ausstoss von Medikamentenflüssigkeit 20 über die selbststeuernden Ventile 7,8;11,12 erzielt. Die Vorrichtung gibt somit in regelmässigen Intervallen, welche voreinstellbar sind und typischerweise zwischen 30 Sekunden und 10 Minuten betragen können, kleine Medikamentendosen ab. Unter Berücksichtigung der relativ langsamen Resorption des Medikamentes durch den menschlichen Körper, ergibt sich ein pseudo-kontinuierlicher Medikamentenfluss.

Fig. 2 zeigt eine alternative Ausführung der Antriebseinheit 15, bei welcher die Kolbenstange 3 mit einem externen Gewinde versehen ist oder eine nicht-rotierbare Spindel 63 mit Aussengewinde trägt und von einem ein Innengewinde aufweisendes Zahnrad 62 umgeben ist. Das Zahnrad 62 greift mit seinen Zähnen in das Zahnrad 64 ein, welches mit dem Schaft der Antriebseinheit 15 gekoppelt ist. Wenn sich der Schaft der Antriebseinheit 15 dreht, dreht sich auch das Zahnrad 62 und die im Eingriff stehenden Gewinde bewirken eine axiale Verschiebung der Spindel 63 in der Art einer Mutter, so dass die Kolbenstange 3 und die damit verbundenen Kolben, welche die Medikamentenabgabe bewirken, mitbewegt werden.

## Patentansprüche

1. Vorrichtung zur Abgabe einer vorwählbaren Dosis eines flüssigen Medikamentes mit einem Medikamentenbehälter (10), wobei die Vorrichtung folgendes umfasst
A) einen Zylinder (1;2) mit einem linken Zylinderteil (1) und einem rechten Zylinderteil (2), einem darin verschieblich angeordneten Doppelkolben (4;5) mit einem linken Kolbenteil (4) und einem rechten Kolbenteil (5) mit Mitteln (6) zur form- und/oder kraftschlüssigen Kupplung an eine Antriebseinheit (15), wobei der linke Kolbenteil (4) und der rechte Kolbenteil (5) über eine Kolbenstange (3) fest miteinander verbunden sind und die beiden Kolbenteile (4;5) gleichsinnig in den beiden Zylinderteilen (1;2) verschieblich angeordnet sind;
B) ein linkes Einlassventil (7) für den linken Zylinderteil (1) und ein rechtes Einlassventil (8) für den rechten Zylinderteil (2), wobei die beiden Einlassventile (7;8) über eine Einlassleitung (9) an einen Medikamentenbehälter (10) anschliessbar sind; und
C) ein linkes Auslassventil (11) für den linken Zylinderteil (1), und ein rechtes Auslassventil (12) für den rechten Zylinderteil (2), wobei die beiden Auslassventile (11;12) über eine Auslassleitung (13) das flüssige Medikament abgeben;
dadurch gekennzeichnet, dass
D) die aufgrund einer durch leichte Überdimensionierung des elastischen Ventil-Materials erreichten Gummivorspannung selbstschliessend wirkenden Einlassventile (7;8) und Auslassventile (11;12) allein durch den hydraulischen Druck, bzw. Unterdruck des flüssigen Medikamentes in passiver Weise steuerbar sind.

2. Vorrichtung nach Anspruch 1, dadurch gekennzeichnet, dass die Mittel (6) zur form- und/oder kraftschlüssigen Kupplung an eine Antriebseinheit (15) an der Kolbenstange (3) angebracht sind.

3. Vorrichtung nach Anspruch 1 oder 2, dadurch gekennzeichnet, dass die an der Kolbenstange (3) angebrachten Mittel (6) zur form- und/oder kraftschlüssigen Kupplung an eine Antriebseinheit (15) aus einer Mitnehmerscheibe (61) bestehen.

4. Vorrichtung nach Anspruch 1 oder 2, dadurch gekennzeichnet, dass die an der Kolbenstange (3) angebrachten Mittel (6) zur kraftschlüssigen Kupplung an eine Antriebseinheit (15) eine nicht-rotierbare Spindel (63) mit Aussengewinde umfasst, welche von einem ein Innengewinde aufweisenden axial festen Zahnrad (62) umgeben ist.

5. Vorrichtung nach einem der Ansprüche 1 - 4, dadurch gekennzeichnet, dass die einzelnen Bauelemente (1-13) zu einer Pumpeneinheit (14) zusammengeschlossen sind, welche in lösbarer Weise an eine Antriebseinheit (15) koppelbar ist.

6. Vorrichtung nach Anspruch 5, dadurch gekennzeichnet, dass die Pumpeneinheit (14) aus gespritzten Kunststoffteilen besteht.

7. Vorrichtung nach einem der Ansprüche 1 - 6, dadurch gekennzeichnet, dass sie mittels einer Aufsteckkappe (18) irreversibel gegen eine patientenseitige Entnahme von Medikamentenflüssigkeit (20) aus dem Medikamentenbehälter (10) schützbar ist.

## Claims

1. Device for administering doses of liquid medicine of predetermined quantity with a medicine container (10), whereby the device comprises
A) a cylinder (1;2) with a left cylinder section (1) and a right cylinder section (2), a double-ended piston (4;5) arranged displaceable therein and having a left piston section (4) and a right piston section (5) with means (6) for coupling by means of positive locking and/or friction connection to a drive unit (15), whereby the left piston section (4) and the right piston section (5) are mutually rigidly connected by means of a piston rod (3) and both piston sections (4;5) are arranged displaceable in same direction within the two cylinder sections (1;2);
B) a left intake valve (7) for said left cylinder section (1 ) and a right intake valve (8) for said right cylinder section (2) whereby said intake valves (7;8) being connectable by an intake line (9) to a medicine container (10); and
C) a left discharge valve (11) for the left cylinder section (1), and a right discharge valve (12) for the right cylinder section (2), whereby said discharge valves (11;12) supplying the liquid medicine through a discharge line (13),
characterized in that
D) said intake valves (7;8) and said discharge valves (11;12) acting self-closing due to a rubber initial tension generated through a slight oversizing of the elastic valve material therewith being passively controllable solely through hydraulic pressure, respectively depression of the liquid medicine.

2. Device according to claim 1, characterized in that said means (6) for coupling to a drive unit (15) by means of positive locking and/or frictional connection being attached to the piston rod (3).

3. Device according to claim 1 or 2, characterized in that said means (6) for coupling to a drive unit (15) by means of positive locking and/or frictional connection attached to the piston rod (3) consist of a drive disk (61).

4. Device according to claim 1 or 2, characterized in that said means (6) for coupling to a drive unit (15) by means of frictional connection comprise a non-rotatable spindle (63) having an external thread being encompassed by a axially fixed gear (62) having an internal thread.

5. Device according to one of the claims 1 - 4, characterized in that the members (1-13) being composed to a pump unit (14), which is loosenably connectable to a drive unit (15).

6. Device according to claim 5, characterized in that the pump unit (14) consists of injection-moulded synthetic parts.

7. Device according to one of the claims 1 - 6, characterized in that it is irreversibly securable against a patient's removal of liquid medicine (20) from said medicine container (10) by means of a snap-on cap (18).

## Revendications

1. Dispositif d'émission d'une dose sélectionnable préalablement d'un médicament liquide, comprenant un récipient à médicament (10), le dispositif comportant
A) un cylindre (1 ; 2) présentant une partie gauche (1) de cylindre et une partie droite (2) de cylindre, un double piston (4 ; 5) disposé de manière à pouvoir y coulisser, et présentant une partie gauche (4) de piston et une partie droite (5) de piston, des moyens (6) d'accouplement en correspondance géométrique ou en correspondance mécanique à une unité d'entraînement (15), la partie gauche (4) de piston et la partie droite (5) de piston étant solidarisées l'une de l'autre par une tige de piston (3) et les deux parties (4 ; 5) de piston étant disposées de manière à coulisser dans le même sens dans les deux parties (1 ; 2) du cylindre ;
B) une soupape gauche d'entrée (7) pour la partie gauche (1) du cylindre et une soupape droite d'entrée (8) pour la partie droite (2) du cylindre, les deux soupapes d'entrée (7; 8) pouvant être reliées à un récipient à médicament (10) par un conduit d'entrée (9) ; et
C) une soupape gauche de sortie (11) pour la partie gauche (1) du cylindre et une soupape droite de sortie (12) pour la partie droite (2) du cylindre, les deux soupapes de sortie (11 ; 12) délivrant le médicament liquide par un conduit de sortie (13) ;
caractérisé en ce que
D) la précontrainte exercée par un léger surdimensionnement sur le matériau de caoutchouc des soupapes d'entrée (7 ; 8) et des soupapes de sortie (11 ; 12) qui se ferment d'elles-mêmes commande les soupapes de manière passive par la pression ou la dépression hydraulique du médicament liquide.

2. Dispositif selon la revendication 1, caractérisé en ce que les moyens (6) d'accouplement en correspondance géométrique ou en correspondance mécanique sont installés sur une unité d'entraînement (15) prévue sur la tige de piston (3).

3. Dispositif selon la revendication 1 ou 2, caractérisé en ce que les moyens (6) d'accouplement en correspondance géométrique et/ou en correspondance mécanique à une unité d'entraînement (15) qui sont installés sur la tige de piston (3) sont constitués d'un disque d'entraînement (61).

4. Dispositif selon la revendication 1 ou 2, caractérisé en ce que les moyens (6) d'accouplement mécanique à une unité d'entraînement (15) qui sont installés sur la tige de piston (3) comportent une broche (63) non rotative dotée d'un filet extérieur et qui est entourée par une roue dentée (62) fixe axialement qui présente un filet intérieur.

5. Dispositif selon l'une des revendications 1 à 4, caractérisé en ce que les composants individuels (1 à 13) peuvent être raccordés à une unité de pompe (14) qui peut être accouplée de manière libérable à une unité d'entraînement (15).

6. Dispositif selon la revendication 5, caractérisé en ce que l'unité de pompe (14) est réalisée en pièces en matière synthétique injectée sous pression.

7. Dispositif selon l'une des revendications 1 à 6, caractérisé en ce qu'il peut être protégé au moyen d'un bouchon enfichable (18) de manière irréversible contre un enlèvement par le patient de médicament liquide (20) hors du récipient à médicament (10).
